(19) Europäisches Patentamt
European Patent Office
Office européen des brevets

(11) **EP 4 535 022 A2**

(12) **EUROPEAN PATENT APPLICATION**

(43) Date of publication:
**09.04.2025  Bulletin 2025/15**

(21) Application number: **24204540.9**

(22) Date of filing: **03.10.2024**

(51) International Patent Classification (IPC):
**G01R 33/56** (2006.01)   **G01R 33/565** (2006.01)
**G06N 3/045** (2023.01)   **G06T 5/60** (2024.01)

(52) Cooperative Patent Classification (CPC):
**G01R 33/56545; G01R 33/5608; G06N 3/045;
G06T 5/60**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority:  **06.10.2023  JP 2023174423**

(71) Applicant: **FUJI-FILM Corporation
Minato-ku
Tokyo 106-8620 (JP)**

(72) Inventors:
• **SHIRAI, Toru
Tokyo (JP)**

• **AMEMIYA, Tomoki
Tokyo (JP)**
• **YOKOSAWA, Suguru
Tokyo (JP)**
• **KANEKO, Yukio
Tokyo (JP)**
• **SUZUKI, Atsuro
Tokyo (JP)**
• **NISHIO, Keisuke
Tokyo (JP)**
• **MURASE, Takenori
Tokyo (JP)**

(74) Representative: **Dehns Germany Partnerschaft
mbB
Theresienstraße 6-8
80333 München (DE)**

(54) **MAGNETIC RESONANCE IMAGING APPARATUS AND IMAGE PROCESSING METHOD**

(57)    Provided is a technology of ensuring data consistency between an image after a CNN (235) is applied and an image before the CNN is applied with a simple CNN configuration in a case in which Gibbs ringing in an MR image is corrected by using the CNN.

An MRI apparatus (1) includes a ringing correction unit (234) that performs ringing correction by using a CNN, in which the ringing correction unit performs a Fourier transform on an image after the CNN is applied to restore the image to k-space data, combines a region of the k-space data corresponding to a measurement matrix region of the image before the CNN is applied with the measurement data of the image before the CNN is applied to obtain k-space data in which the measurement matrix region is replaced with the measurement data of the image before the CNN is applied. An inverse Fourier transform is performed on the k-space data to obtain a final image.

FIG. 7

EMBODIMENT 1

START

PERFORM ZERO-FILL ON MEASUREMENT IN k-SPACE — S21

APPLY CNN IN REAL SPACE — S22

REPLACE DATA IN MEASUREMENT RANGE AFTER CNN IS APPLIED WITH ORIGINAL MEASUREMENT DATA IN k-SPACE (Data Consistency PROCESSING) — S23

TRANSFORMATION INTO REAL SPACE — S24

END

EP 4 535 022 A2

**Description**

**CROSS-REFERENCE TO RELATED APPLICATIONS**

The present application claims priority under 35 U.S.C. § 119 to Japanese Patent Application No. 2023-174423, filed October 6, 2023. Each of the above application(s) is hereby expressly incorporated by reference, in its entirety, into the present application.

**BACKGROUND OF THE INVENTION**

1. Field of the Invention

The present invention relates to a magnetic resonance imaging apparatus, and more particularly, to a ringing correction technology of an image acquired by the magnetic resonance imaging apparatus.

2. Description of the Related Art

In magnetic resonance imaging (MRI), a nuclear magnetic resonance signal (echo signal) applied with encoding in two-axial directions in a case of two-dimensional measurement or encoding in three-axial directions in a case of three-dimensional measurement is generated to acquire measurement data that is two-dimensional or three-dimensional k-space data. The MRI apparatus reconstructs an image of the subject by performing processing such as a Fourier transform on the measurement data.

A matrix size (measurement matrix size) and a data pattern for filling the k-space (sampling pattern) during measurement are determined by a pulse sequence used for imaging, but vary depending on imaging conditions such as restrictions of the apparatus and an imaging time.

On the other hand, in the reconstruction, the measurement data in a frequency domain is transformed into image data in a real space domain by performing an inverse Fourier transform, so the measurement data is processed as rectangular or rectangular parallelepiped data in terms of the operation of the inverse Fourier transform. In addition, the matrix size of the image to be reconstructed (reconstruction matrix size) may be the same as the measurement matrix size, but in order to obtain an image with a desired resolution, the image is often reconstructed with a matrix size different from the measurement matrix size.

Since the measurement matrix size is discrete and finite in a frequency space, in a case in which the measurement matrix size and the reconstruction matrix size are different from each other and a ratio thereof deviates from 1:1, a periodic or streak artifact called a Gibbs ringing or a truncation artifact (hereinafter referred to as ringing) is more strongly generated in the reconstructed image.

As a technology of reducing a noise or an artifact included in an image obtained by the MRI, various technologies using neural networks have been proposed, and, for example, Qianqian Zhang et al. Magn Reson Med. 2019;82:2133-2145 proposes using a deep learning model, such as a CNN, which has been trained by using images reconstructed by combining various measurement matrix sizes and reconstruction matrix sizes, to reduce an image artifact.

However, in a case in which the CNN is applied to correct the ringing, there is a problem in that the measurement data of a measurement matrix region (region from which the signal is actually obtained) as a source of an input image to the CNN is changed. In a case in which the measurement data is changed, a structural change may be caused on the image reconstructed from the measurement data.

In the image processing technology using the neural network, a technology of maintaining consistency between the data in a case in which the neural network is applied and the input data to prevent changes in the data after processing has also been proposed. For example, JP2021-532875A discloses a method of providing a data consistency layer in a neural network and not changing a value of spatial frequency data (k-space data) obtained by an MRI apparatus.

**SUMMARY OF THE INVENTION**

In a case in which the Gibbs ringing correction is performed on the MR image, images as correction targets have various conditions such as a size of the measurement matrix region, particularly a ratio to the reconstruction matrix size, and a scanning method (sampling pattern) during data measurement, and a large amount of training data is required for training the CNN for the ringing correction corresponding to images with different conditions, and thus it is necessary to construct a simple CNN. In the method described in JP2021-532875A, in order to maintain the data consistency after the CNN is applied, the data consistency layer combined with a non-uniform (non-equidistant) Fourier transform is adopted, but the construction of CNN is complicated, making it difficult to train the CNN corresponding to the images with different conditions.

An object of the present invention is to provide a technology capable of ensuring data consistency with a simple configuration and effectively performing ringing correction while maintaining original measurement data.

In order to achieve the above-described object, in the present invention, ringing correction using a CNN is performed, and then processing of replacing a measurement matrix region of pre-processed data with data after the CNN processing in the measurement space is performed on the image after the CNN is applied, thereby ensuring data consistency.

That is, an aspect of the present invention provides an MRI apparatus comprising: an imaging unit that collects measurement data consisting of nuclear magnetic resonance signals; an image generation unit that has a function of reconstructing the measurement data at a desired reconstruction matrix size; and a ringing correction unit that corrects ringing that occurs in a reconstructed image in a case in which a measurement matrix size of the measurement data and the reconstruction matrix size are different from each other.

In the aspect of the present invention, the ringing correction unit includes one or a plurality of CNNs that have been trained to generate an output image in which Gibbs ringing of an input image is corrected, and a combining unit that generates composite data by replacing a part of k-space data obtained by performing a Fourier transform on the output image of the CNN with the measurement data before the input image of the CNN is reconstructed, the ringing correction unit performing an inverse Fourier transform on the composite data to generate a ringing-corrected image.

In addition, in another aspect of the present invention, the ringing correction unit includes one or a plurality of CNNs that have been trained to generate an output image in which the Gibbs ringing of the input image is corrected, and the CNN includes a CNN that has been trained by using, as training data, a correct answer image in which the ringing has not occurred and an image obtained performing an inverse Fourier transform on k-space data in which a size of a measurement matrix region is smaller than a matrix size of the correct answer image, and increasing a weight of an error in the measurement matrix region that to be larger than a weight of an error in a region other than the measurement matrix region in a loss evaluation function during training.

Another aspect of the present invention provides an image processing method of, in a case in which measurement data consisting of nuclear magnetic resonance signals collected by an MRI apparatus is reconstructed at a desired reconstruction matrix size, correcting ringing that occurs in a reconstructed image in a case in which a measurement matrix size of the measurement data and the reconstruction matrix size are different from each other.

The image processing method comprises a step of applying one or a plurality of CNNs that have been trained to generate an output image in which the ringing of an input image is corrected, to a correction target image; a step of generating k-space data by performing a Fourier transform on the correction target image before the CNN is applied and a corrected image after the CNN is applied; a step of generating composite k-space data by replacing a part of the k-space data of the corrected image with a measurement matrix region in the k-space data of the correction target image; a step of performing an inverse Fourier transform on the composite k-space data.

According to the aspect of the present invention, by performing the ringing correction using the CNN and then combining the corrected image with the image before the CNN is applied in the measurement space, it is possible to maintain the data consistency, significantly reduce a load of constructing the CNN, and it is possible to effectively perform the ringing correction on the measurement data having various matrix ratios and sampling patterns using a simple configuration. In addition, since the combining unit performs only the low-load operations such as the Fourier transform and the data replacement, a unit that performs the image processing can be configured simply.

In addition, by repeating the CNN processing and the combining processing corresponding to data consistency processing, the improvement of the accuracy of the ringing correction and the improvement of the image quality can be achieved.

Further, according to a second aspect of the present invention, during the training of the CNN, by applying weighting to the error of the Loss function and increasing the weight of the error in the measurement matrix region to be larger than the weight of the error in the region other than the measurement matrix region, the function of the data consistency can be provided without changing the structure of the CNN itself.

## BRIEF DESCRIPTION OF THE DRAWINGS

Fig. 1 is a diagram showing an overall configuration of an MRI apparatus to which the present invention is applied.
Fig. 2 is a functional block diagram of an image processing unit.
Fig. 3 is a diagram showing an outline of an image processing procedure.
Fig. 4 is a diagram showing an example of a structure of a CNN adopted in Embodiment 1.
Fig. 5 is a diagram showing training data for the CNN.
Fig. 6 is a diagram showing a training process of the CNN.
Fig. 7 is a diagram showing a flow of ringing correction of Embodiment 1.
Fig. 8 is a diagram showing the ringing correction including DC processing.
Fig. 9 is a diagram showing a flow of ringing correction of Embodiment 2.
Fig. 10 is a diagram showing a relationship between a measurement matrix size and a reconstruction matrix size.
Fig. 11 is a diagram showing the occurrence of Gibbs ringing.
Fig. 12 is a diagram showing processing of Application Example 1.
Fig. 13 is a diagram showing a training image set for a CNN in Application Example 2.
Fig. 14 is a diagram showing image processing of Application Example 2.
Fig. 15 is a diagram showing ringing correction processing of Application Example 3.

Fig. 16 is a diagram showing an application example to an elliptical sampling pattern.

## DESCRIPTION OF THE PREFERRED EMBODIMENTS

Hereinafter, embodiments of an MRI apparatus and an image processing method realized by the MRI apparatus according to an embodiment of the present invention will be described with reference to the accompanying drawings.

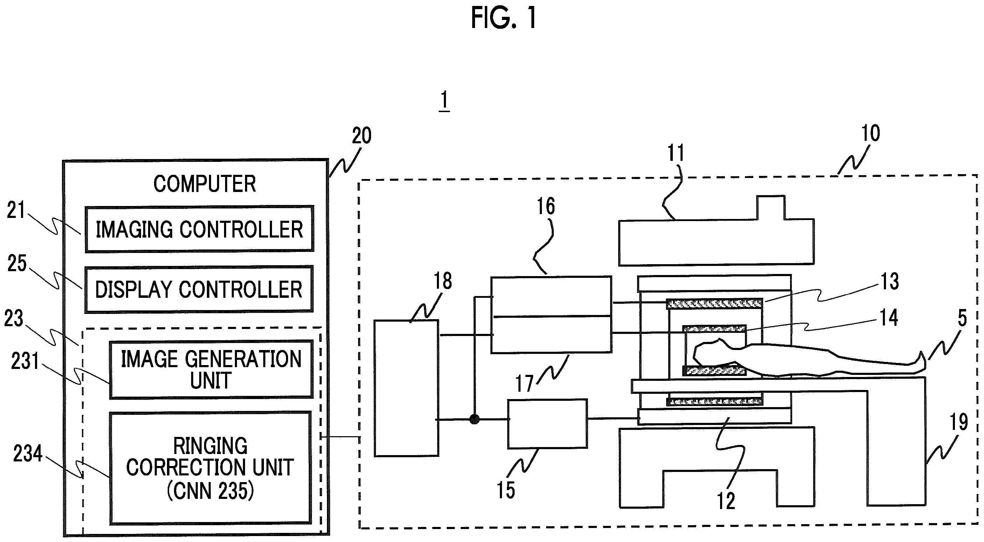

First, an outline of a configuration of an MRI apparatus 1 to which the present invention is applied will be described. The present invention can be applied to most types of MRI apparatuses that are currently in widespread use, and the configuration thereof roughly includes an imaging unit 10 that collects nuclear magnetic resonance signals, a computer 20 that controls the imaging unit and performs various types of operations, and various devices that are associated with the imaging unit 10 and the computer 20, such as an input device, a display device, and an external storage device, as shown in Fig. 1.

Since the configuration of the imaging unit 10 is the same as a configuration of a normal MRI apparatus, a detailed description thereof will be omitted, but the imaging unit 10 comprises a static magnetic field magnet 11 that generates a static magnetic field space in which a subject 5 is placed, a gradient magnetic field coil 12 that applies a gradient magnetic field in a static magnetic field, an RF transmission coil 13, an RF receive coil 14, a gradient magnetic field power supply 15 that drives these coils and a transmitter 16, a receiver 17 to which a high-frequency receive coil is connected, and a bed device 19 that transports the subject 5 into the static magnetic field space. Further, the imaging unit comprises a sequencer 18 that controls the operations of sampling the signals via the gradient magnetic field coil 12, the RF transmission coil 13, and the RF receive coil 14 in accordance with a pulse sequence.

The computer 20 includes an imaging controller 21 that controls the operation of the imaging unit 10 via the sequencer 18, an image processing unit 23 that performs image reconstruction and other processing on measurement data (k-space data) collected by the imaging unit 10, and a display controller 25 that controls the display of the reconstructed images, a GUI, and the like.

The computer 20 can be configured as a general-purpose computer comprising a CPU or a GPU and a memory, and the above-described functions of the computer are programmed in advance and realized by uploading the program to the CPU or the like. In addition, some functions of the computer may be realized by hardware such as an ASIC or a programmable IC, and the computer according to the embodiment of the present invention includes these hardware components.

The image processing unit 23 according to the present embodiment includes an image generation unit 231 and a ringing correction unit 234.

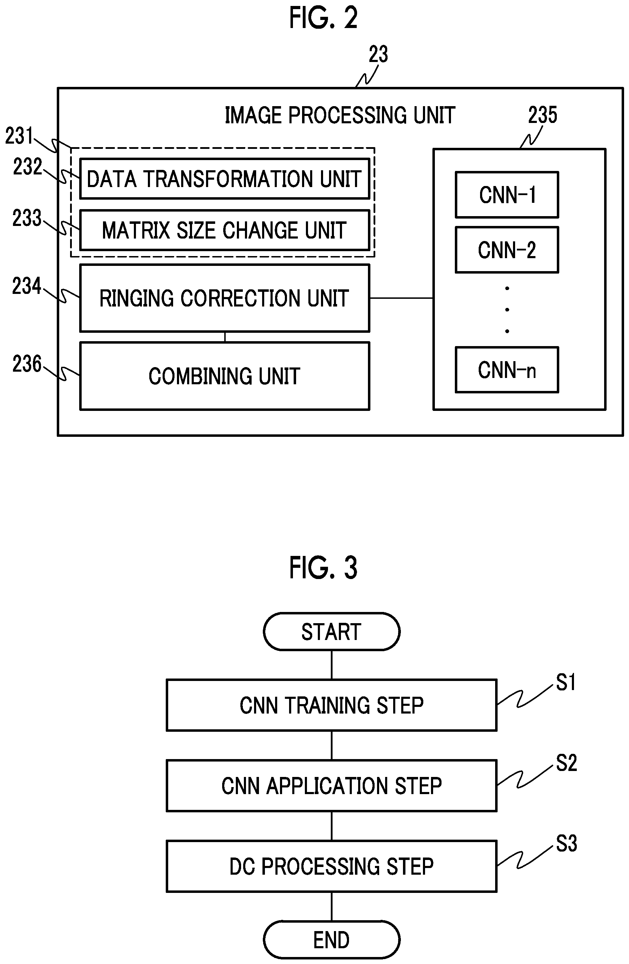

The image generation unit 231 is a functional unit that performs operations such as an inverse Fourier transform using measurement data collected by the imaging unit 10, that is, k-space data, to reconstruct an image, and comprises, as shown in Fig. 2, a data transformation unit 232 that transforms the measurement data (k-space data) into real space data (image data), and a matrix size change unit 233 that changes a matrix size of the k-space data.

The ringing correction unit 234 comprises one or a plurality of CNNs 235 that have been trained to correct Gibbs ringing that has occurred in a reconstructed image, and a combining unit 236 that combines k-space data of the image after the CNN 235 is applied with the original measurement data to perform processing of maintaining data consistency.

As shown in Fig. 3, the image processing unit 23 having the above-described configuration executes a step (S 1) of training the CNN 235 by using a correct answer image in which the ringing has not occurred or the ringing has occurred to the extent that the ringing can be ignored and an input image (input image for training) reconstructed under a condition in which the ringing has occurred, a step (S2) of applying the trained CNN 235 to the image reconstructed by the image generation unit 231 to correct the ringing of the image, and a step (S3) of combining the k-space data of the output image of the CNN 235 and the k-space data of the image before input to the CNN 235 by the combining unit 236. As a result, the measurement data changed by the ringing correction via the CNN is replaced with the original measurement data, allowing the final image, which has undergone the ringing correction while preserving the information of the original measurement data, to be obtained.

Hereinafter, a specific embodiment of processing performed by the image processing unit 23 will be described. In the following description, Fig. 2 showing a configuration example of the image processing unit 23 and Fig. 3 showing an outline of the processing will be referred to as needed.

Embodiment 1

In the present embodiment, the ringing correction of an image (two-dimensional image or three-dimensional image) in which the measurement matrix of the measurement data is a rectangular or rectangular parallelepiped shape, the matrix size is expanded in a two-dimensional direction or a three-dimensional direction, and the image is reconstructed is described as an example. In addition, in the drawings referred to, a case in which a two-dimensional image is used as a target is shown for simplicity of the description.

First, a CNN that has been trained to correct ringing that has occurred in the image is prepared (Fig. 3:

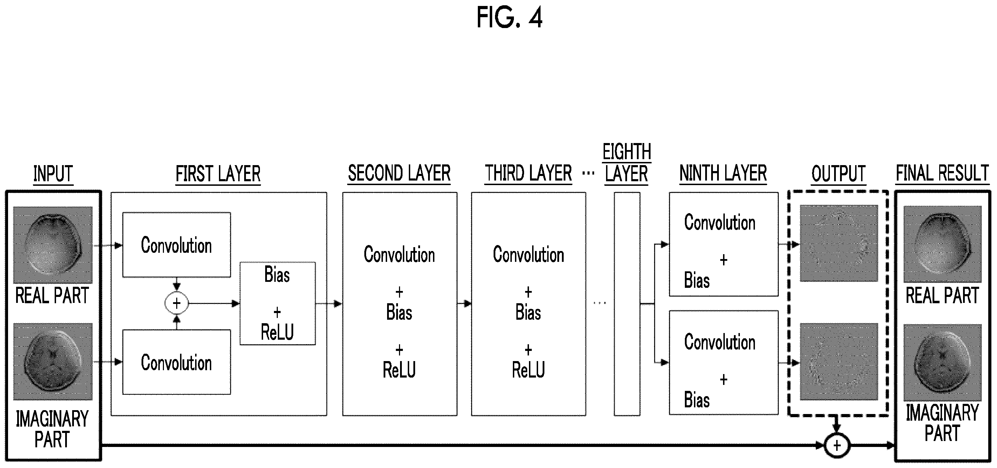

S1). Since the measurement data obtained by the MRI apparatus is complex data and the image is also a complex image, the CNN is a complex-valued CNN. Fig. 4 shows an example of the complex-valued CNN adopted in the present embodiment.

The CNN 235 is a complex-valued CNN having a nine-layer structure in which each layer is formed by a block consisting of a convolution layer, an activation function (ReLU), and a pooling layer, the real part and the imaginary part of the input image are combined in the first layer, and the real part and the imaginary part are separated in the ninth layer. A kernel size or the number of channels of each layer is not particularly limited, but for example, the kernel size can be about $3 \times 3$ to $9 \times 9$, and the number of channels can be about 16 Ch to 128 Ch. In addition, in the present embodiment, a simple CNN is shown as an example, but the CNN is not limited to this. For example, a resolution-changed CNN, such as U-net, may be used.

Training Step of CNN: S1 of Fig. 3

The CNN is trained by using a large number of training data sets of training data including an image (correct answer image) acquired under a condition in which Gibbs ringing has not occurred or the Gibbs ringing has occurred to the extent that the Gibbs ringing can be ignored and an image (input image for training) obtained under a condition in which Gibbs ringing has occurred for the same imaging target, and repeating processing of adjusting parameters (weights and biases) of the neural network such that the output image in which a difference between the input image and the correct answer image is minimized is obtained.

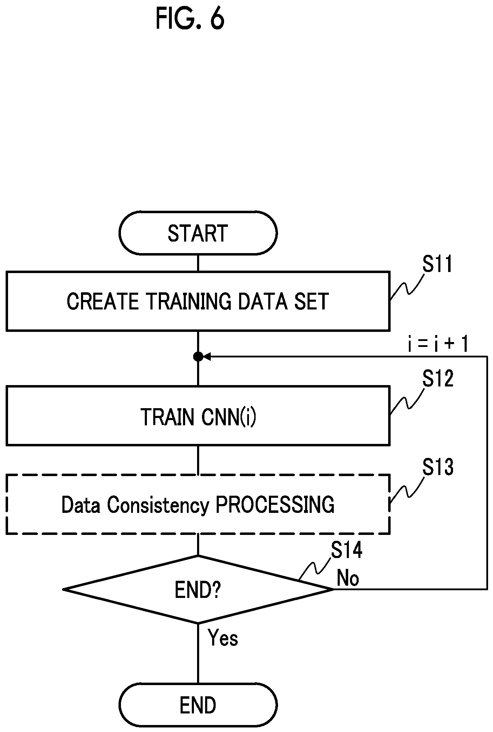

Therefore, first, the correct answer image and the input image for training are prepared as the training data (Fig. 6: S11). As the correct answer image, an image reconstructed from the measurement data (two-dimensional or three-dimensional k-space data) captured by three-dimensional measurement at a large measurement matrix size is used. In a case in which the measurement matrix size is equal to or larger than the reconstruction matrix, almost no Gibbs ringing occurs in the reconstructed image.

The input image for training is an image in which the ringing has occurred, and for example, an image reconstructed by performing zero-filling of a high-frequency region on measurement data obtained at a measurement matrix size smaller than the above-described measurement data to change a size of the measurement data to the k-space data having the same matrix size as the k-space data of the correct answer image.

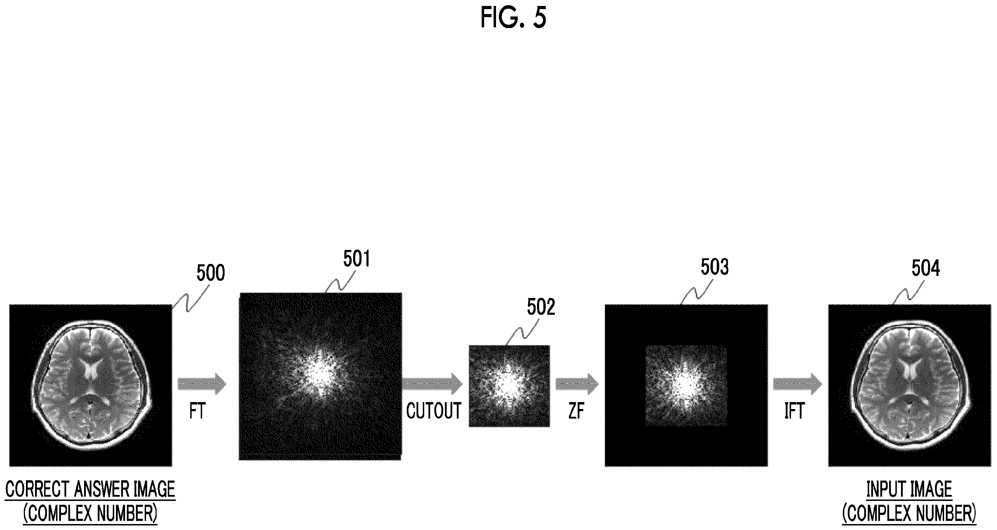

Fig. 5 shows an example of the training data used for training the CNN. In this example, correct answer image 500 is subjected to a Fourier transform (FT) to obtain k-space data 501, a low-frequency region of the k-space data 501 is cut out, and the periphery (high-frequency region) of cutout data 502 is subjected to zero-filling (ZF), and then k-space data 503 after zero-filling is subjected to an inverse Fourier transform (IFT). An image 504 obtained by this way is used as the input image for training. It should be noted that the example shown in Fig. 5 is a representative example of the training data, and the present invention is not limited thereto.

For example, in the present embodiment, as an example, a case is described in which the measurement data of the rectangular sampling pattern is expanded in the two-dimensional direction and the size is changed as described above, but the measurement matrix size of the input image for training, the sampling pattern during measurement, and the expansion direction of the matrix (axial direction in which the size is changed), so that it is also possible to generate the trained CNN corresponding thereto. The modification example of the training data will be described in detail in the embodiments and the application examples described below.

In the training of the CNN, as shown in Fig. 6, the parameters of the CNN are repeatedly adjusted by using the prepared correct answer image and the input image for training until the difference (Loss evaluation function) between the input image after the CNN is applied and the correct answer image is minimized (S12, S14). For each repetition, the input image of the CNN is updated with the output image of the previous repetition. Here, the image for updating the input image may be the output image of the immediately preceding repetition, but as shown by a dotted line in Fig. 6, the output image may be subjected to data consistency processing (hereinafter referred to as DC processing) described later (S13), and the processed image may be used as the input image for the next repetition. In this way, by adding the DC processing in the training process of the CNN, the ringing correction accuracy of the CNN can be improved.

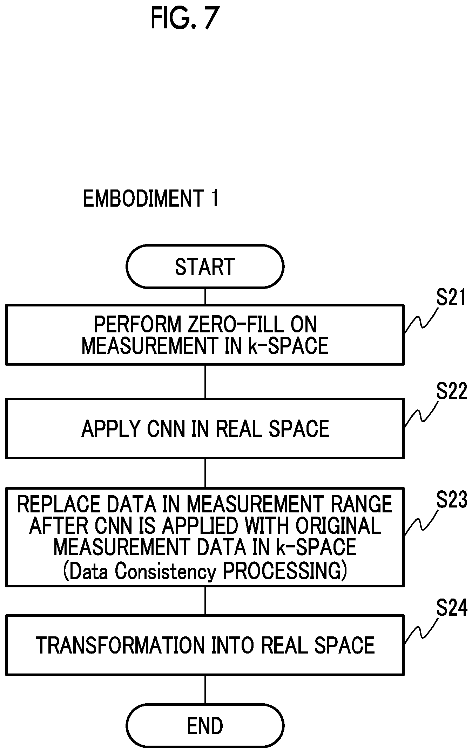

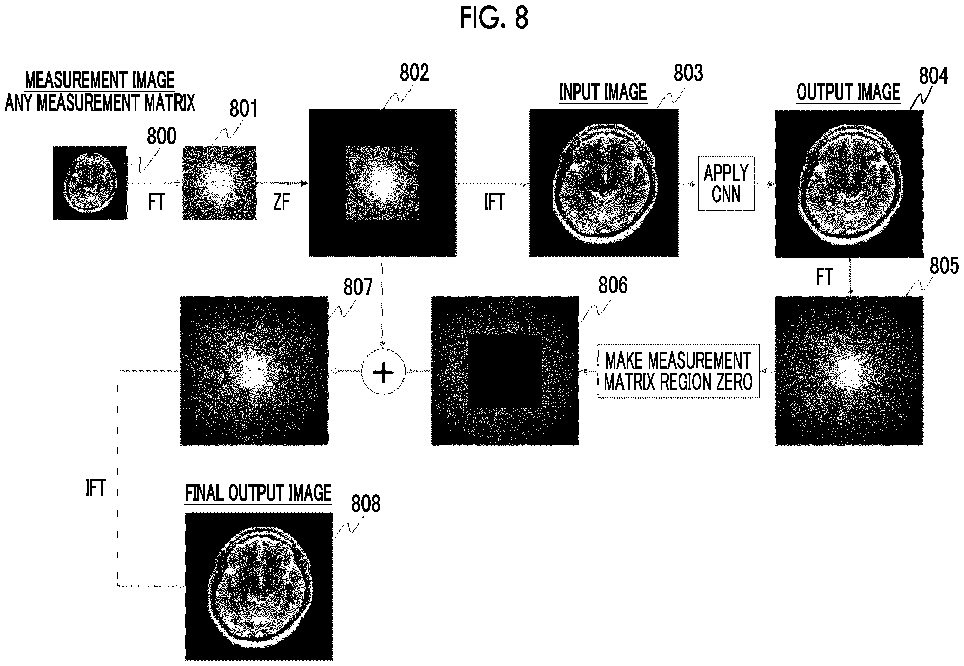

Hereinafter, the ringing correction processing (S2 in Fig. 3) using the trained CNN 235 and the processing (including the DC processing) (S3 in Fig. 3) performed by the combining unit 236 will be described with reference to Figs. 7 and 8.

Ringing Correction: S21, S22

The CNN 235 is applied to the image that is the ringing correction target. As shown in Fig. 8, the image to which the CNN 235 is applied is an image 800 in which the measurement data collected by the imaging unit 10 at any measurement matrix size is reconstructed at the reconstruction matrix size different from the measurement matrix size. Specifically, as shown in Fig. 8, the image is an image 803 obtained by expanding the matrix size of the measurement data 801 of the image 800 acquired by the imaging unit 10 by performing zero-filling (ZF) of the periphery thereof in accordance with the reconstruction matrix size (S21), and performing an inverse Fourier transform (IFT) on the expanded k-space data 802. The Gibbs ringing has occurred in the image 803 in the direction in which the matrix size is changed, for example, in the two-dimensional direction in a case of a two-dimensional size change. By inputting the image to the trained CNN, to obtain an output image 804 in which the ringing is corrected (S22).

DC processing: S23

The combining unit 236 performs the DC processing on the output image 804 of the CNN 235. As shown in Fig. 8, in the DC processing, the output image 804 is subjected to a Fourier transform to be restored to k-space data 805, a region 806 corresponding to the measurement matrix size of the measurement data 801 is set to zero, and the original measurement data 801 is added to the region 806. That is, the k-space data 805 in which the region 806 is set to zero and the k-space data 802 after zero-filling are combined to obtain combined k-space data 807.

Finally, the combined k-space data 807 is subjected to an inverse Fourier transform (IFT) to obtain a final image 808 (S24).

According to the present embodiment, by performing the DC processing after the CNN is applied, the original measurement data is maintained in the final image, and the high-frequency region is extrapolated, thereby suppressing the ringing.

Embodiment 2

In the present embodiment, the two types of processing in Embodiment 1, that is, the ringing correction processing using the CNN 235 and the DC processing via the combining unit 236, are repeated two or more times.

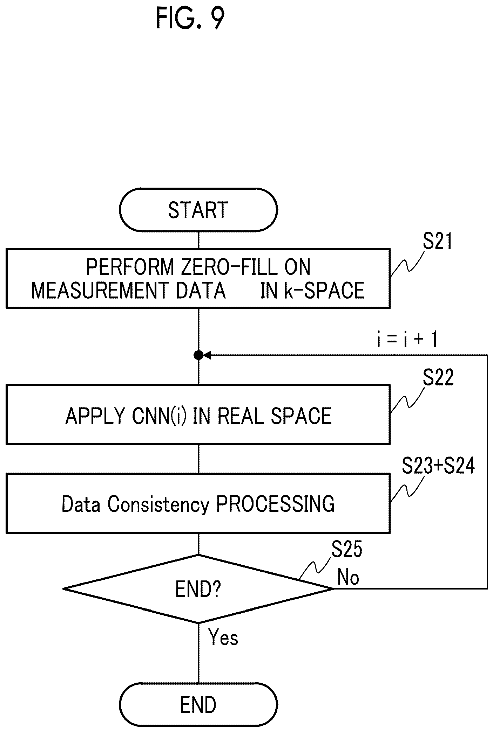

Fig. 9 shows a flow of processing according to the present embodiment. The specific contents of the training process of the CNN 235 and the subsequent processing are the same as the specific contents in Embodiment 1, and thus the overlapping description is omitted.

In the present embodiment as well, the measurement data of the image, which is the correction target, is expanded the zero-filling to have the desired reconstruction matrix size (S21), and the k-space data after zero-filling is subjected to an inverse Fourier transform to obtain image data, and the CNN 235 is applied (S22). Next, the combining unit 236 replaces the low-frequency region of the k-space data of the output image of the CNN 235 with the original measurement data, and combines the k-space data in which the low-frequency region is the original measurement data and the high-frequency region surrounding the low-frequency region is the data after the CNN is applied (S23). After these two types of processing end, the combined k-space data is subjected to an inverse Fourier transform to obtain image data (S24), and the processing is returned to the ringing correction processing using the CNN 235, these types of processing (S22 to S24) are repeated. In a determination of whether or not to end the processing (S25), a predetermined number of repetitions may be set in advance, or the determination may be made by using an evaluation function such as an error function. In addition, in a case in which the CNN 235 consists of a plurality of CNNs having different characteristics and the plurality of CNNs can be applied in multiple stages in accordance with the characteristics of the measurement data, a different CNN may be applied for each repetition.

According to the present embodiment, by repeating the ringing correction processing and the DC processing within a limited range, a ringing correction effect can be further enhanced.

Embodiment 3

The present embodiment is characterized in that the ringing correction processing (Fig. 3: S2) and the DC processing (Fig. 3: S3) are the same as those in Embodiment 1 or Embodiment 2, and the effect of the DC processing is incorporated in the training of the CNN.

As described in Embodiment 1, in the training of the CNN, the parameters such as the weights and the biases of the CNN are adjusted while updating the input image, and the training end is determined by using the Loss evaluation function. A known function such as the sum of squares of the difference is used as the Loss evaluation function, but in the present embodiment, the CNN is trained by increasing, in the loss evaluation function, the weight of the error in the measurement matrix region, that is, the matrix region of the actually measured measurement data, to be larger than the weight of the error in other regions.

Specifically, in a case in which the correct answer image (full-sampling image) is denoted by A, the k-space data is denoted by ka, the input image (zero-filled image) is denoted by B, and the k-space data is denoted by kb, in the related art, the weights and the biases of the CNN are trained such that a loss function represented by Equation (1) is minimized.

$$\mathrm{Loss} = |A - B|^{\wedge}2 = |ka - kb|^{\wedge}2 \ (1)$$

On the other hand, in the present embodiment, as shown in Expression (2), a Loss function in which a weight $W(kr)$ (weight at the coordinate kr) is given to the coordinate in the k-space is used, and the weight is varied depending on the region.

$$\mathrm{Loss} = |W(ka - kb)|^{\wedge}2 \ (2)$$

Here, $W(kr)$ is, for example, defined in the following manner.

$W(kr) = 1$ in a case in which Kr is within the measurement matrix region.
$W(kr) = 0.5$ in a case in which Kr is outside the measurement matrix region.

It should be noted that the numerical value of the weight is merely an example and is not limited thereto.

By increasing the weight in the measurement matrix region to be larger than the weight outside the region in this way, the weights and the biases of the CNN are trained such that the signal in the measurement matrix region is not changed, and the outside of the region is extrapolated.

The method of training the CNN according to the present embodiment is different from the technology described in JP2021-532875A in that a specific data consistency layer is not inserted into the CNN, and the CNN itself is trained to maintain the data consistency, so that the learning effect can be obtained with a simple CNN configuration.

Modification Example of Embodiment 3 Second Aspect

In Embodiment 3, as in Embodiment 1, the DC processing is performed by the combining unit 236 after the ringing correction using the CNN, but in Embodiment 3, in a case in which the CNN is trained, the training is performed such that the data consistency is maintained, and thus it is also possible to omit the DC processing (S23) after the CNN is applied.

Although the embodiments of the MRI apparatus and the image processing method according to the embodiment of the present invention are described above, in the present invention, it is also possible to perform the training of the CNN in accordance with the measurement conditions, such as the measurement matrix and the sampling pattern of the image that is the ringing correction target. Hereinafter, an embodiment (application example) will be described in which the ringing correction and the DC processing are performed using a plurality of trained CNNs.

Application Example 1 Matrix Ratio-Fixed CNN

In the present application example, a CNN trained by using training data in which a ratio (reconstruction matrix size/measurement matrix size) of the reconstruction matrix size to the measurement matrix size of the measurement data is a predetermined matrix ratio is used.

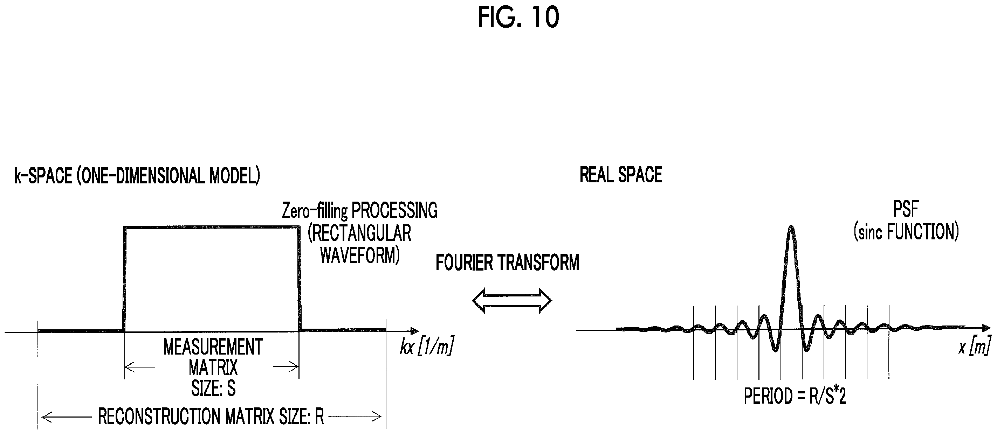

The correct answer image and the input image for training used as the training data are the same as those in Embodiment 1, but all of a large number of training data sets have the same ratio between the matrix size of the correct answer image and the measurement matrix size of the input image for training. The Gibbs ringing has occurred by the presence of a step between the measurement matrix and the high-frequency region around the measurement matrix region due to zero-filling performed on the periphery of the measurement matrix region of the k-space data, as shown in Fig. 10. In addition, it can be known that the width of the streak of the ringing corresponds to the frequency of the point spread function (PSF) (function shown on the right side of Fig. 10) obtained by performing a Fourier transform on a cutout waveform of the measurement data, and depends on the matrix ratio. Therefore, the CNN that has been trained to perform the ringing correction by keeping the matrix ratio constant can obtain a high ringing correction effect at this matrix ratio.

As the matrix ratio of the training data, one or a plurality of representative matrix ratios in the MR image can be adopted. In a case in which the ringing correction unit 234 comprises a plurality of CNNs trained for each of the plurality of matrix ratios, one or two CNNs closest to the matrix ratio of the image that is the correction target are selected from among the plurality of CNNs and applied. In a case of comprising one CNN that has been trained at a predetermined matrix ratio, it is preferable to perform filter processing in addition to the DC processing after the CNN processing. In a case of performing the filter processing, the DC processing is executed, and then the filter processing is performed.

In a case in which an image having a matrix ratio different from the matrix ratio of the training data of the CNN is processed, large ringing is corrected, but small ringing may remain. By adding the filter processing, such residual ringing can be reduced.

According to the present application example, by comprising the CNN that has been trained with a fixed matrix ratio, it is possible to obtain a high ringing correction effect for an image in which the matrix ratio is within a specific range.

Application Example 2 Low-Dimensional Direction Correction Effect CNN

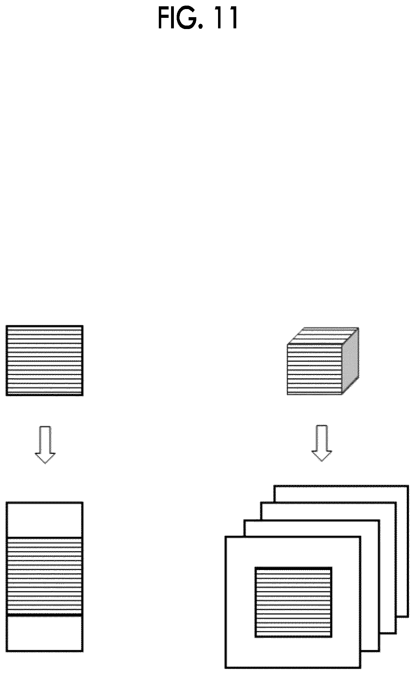

In Embodiment 1, the image expanded in the two-dimensional direction in a case of performing zero-filling on the rectangular measurement data and subjected to an inverse Fourier transform is used as the input image for training the CNN, but it is also possible to train the CNN to obtain a ringing correction effect for the one-dimensional direction by expanding only in the one-dimensional direction instead of the two-dimensional direction. As a result, a trained CNN having a ringing correction effect for the one-dimensional direction is obtained. The same applies to a case of the three-dimensional measurement data, the image obtained by performing an inverse Fourier transform on the k-space data expanded by performing zero-filling in the two-dimensional direction or the one-dimensional direction instead of zero-filling in the three-dimensional direction may be used, and a trained CNN having a ringing correction effect for the expansion direction can be obtained. Fig. 11 shows an example of measurement data obtained by expanding two-dimensional measurement data in a one-dimensional direction and an example of measurement data obtained by expanding three-dimensional measurement data in a two-dimensional direction.

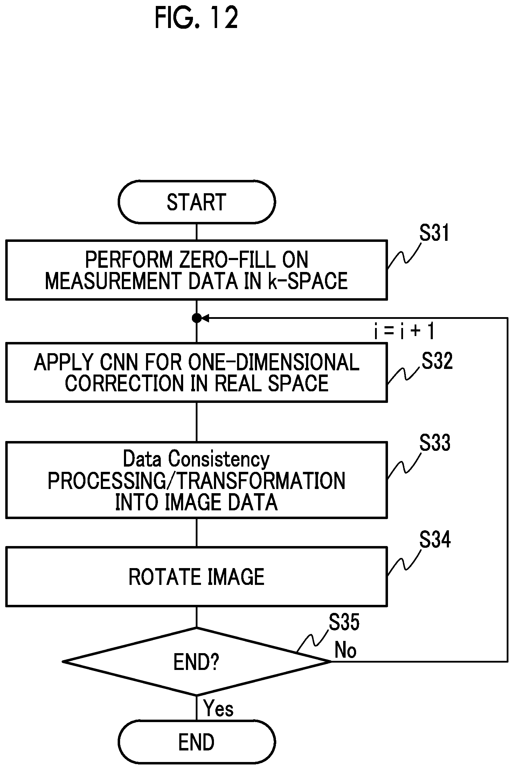

In a case of applying the CNN having such a ringing correction effect for the low-dimensional direction, the ringing correction in accordance with the dimension of the image that is the correction target can be performed by using the CNN in multiple stages. For example, in a case in which the correction target is a two-dimensional image obtained by zero-filling in two directions, as shown in Fig. 12, first, the CNN for the one-dimensional direction correction is applied to perform the ringing correction for the direction (S31, S32), and then the DC processing is performed (S33). Next, the image after the ringing correction and the DC processing is rotated such that the expansion direction of the measurement data matches the one-dimensional direction corrected by the CNN (S34, S35), the CNN for the same one-dimensional correction is applied (S32), and then the DC processing is performed (S33). That is, in the present application example, Embodiment 2 is applied, and the two types of processing are repeated, but the rotation of the image (S34) is added between the repetitions.

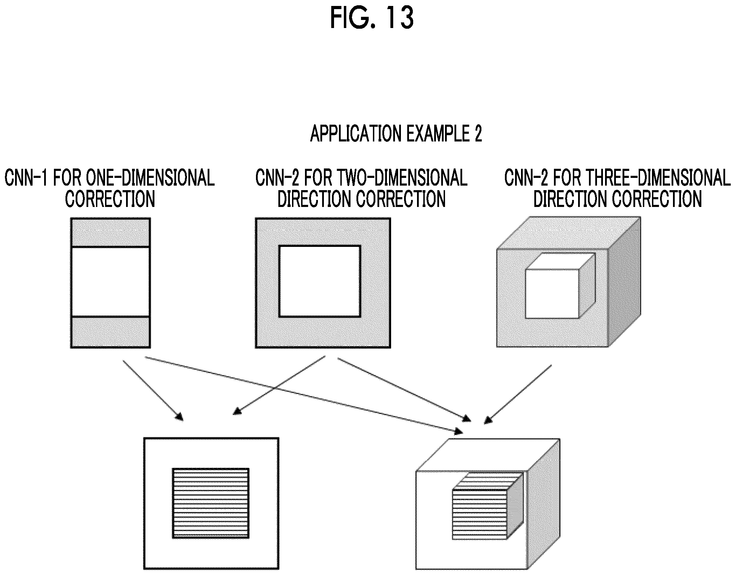

In a case of the three-dimensional image, the CNN for the one-dimensional correction can be repeatedly used three times while interposing processing of matching the correction direction of the CNN with the expansion direction of the matrix size. Alternatively, it is also possible to use the CNN that has learned a ringing correction effect for the two-dimensional direction, apply the CNN for the two-dimensional direction correction to one surface of the three-dimensional data, and then apply the CNN for the one-dimensional correction for a direction orthogonal to the surface in a sequential manner, or to apply the CNN for the one-dimensional correction for one direction and then apply the CNN for the two-dimensional correction. In this case as well, the DC processing and image the transposition processing are performed after one application of the CNN, which is the same as in a case of the two-dimensional image. That is, as shown in Fig. 13, by combining the CNNs for the one-dimensional correction and the two-dimensional correction, it is possible to perform the ringing correction on the image in which the ringing has occurred in more axial directions than the number of axes (the number of directions) learned by the CNN.

According to the present application example, since the CNN for the one-dimensional direction correction or the two-dimensional direction correction need only be trained as the CNN that performs the ringing correction, the burden of the training and the application of the CNN can be reduced, and the same ringing correction effect as in a case of using the CNN trained using an image having the same dimension as the image that is the correction target can be obtained.

It should be noted that the CNN that corrects the ringing in three directions may be applied once to the three-dimensional image.

Application Example 3 For Each Sampling Pattern

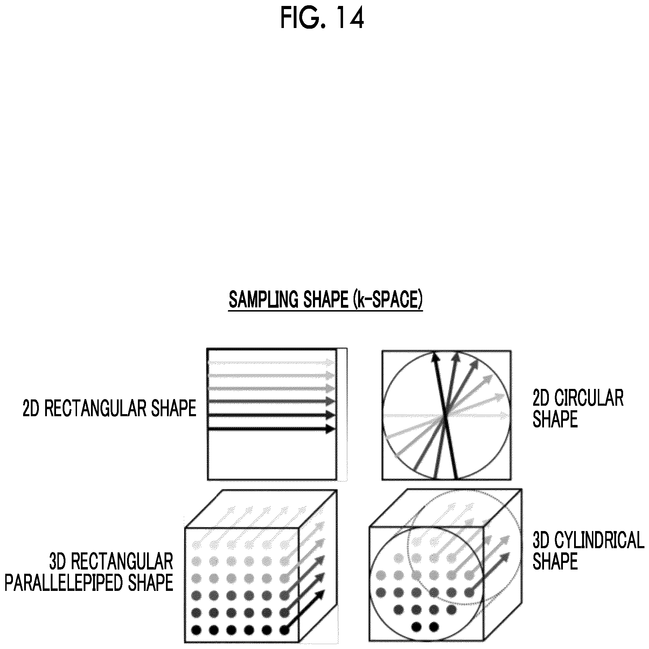

In the present application example, the CNN is trained for each sampling pattern of the measurement data, and the CNN corresponding to the sampling pattern of the correction target image is used. As shown in Fig. 14, for the collection of the measurement data in the MRI apparatus, the raster scanning in which the k-space is scanned parallel to the axis thereof and radial scanning in which the k-space is scanned radially with the origin of the k-space as the center are used. The former is a rectangular sampling pattern in a case of the two-dimensional data and a rectangular parallelepiped sampling pattern in a case of the three-dimensional data. The latter is an elliptical sampling pattern in a case of the two-dimensional data and a cylindrical or spherical sampling pattern in a case of the three-dimensional data.

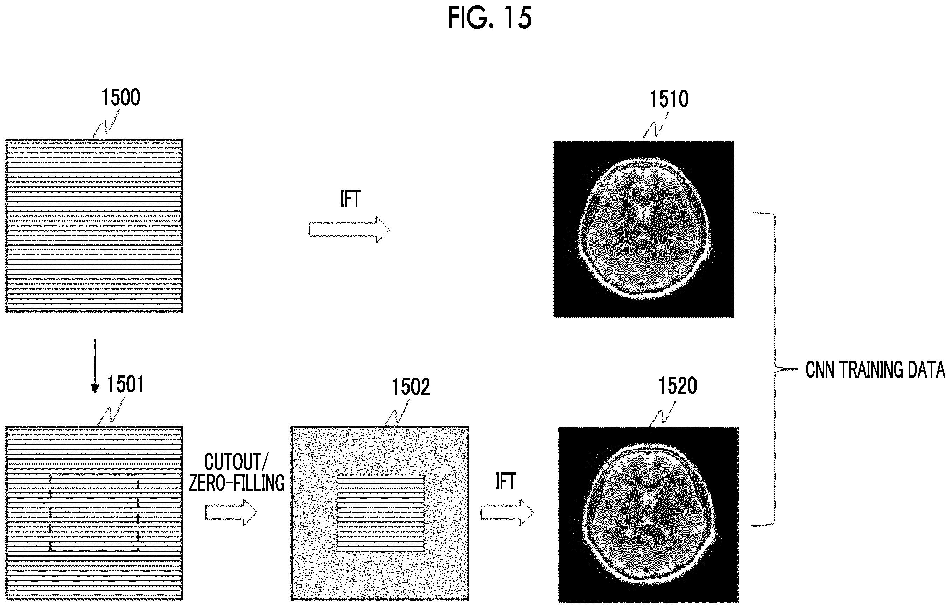

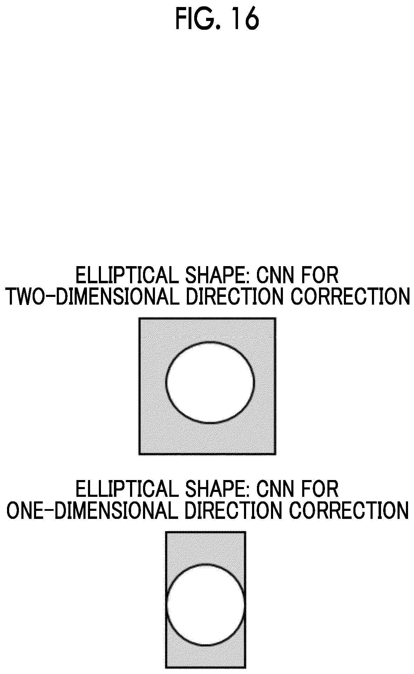

In the present application example, the CNN is trained by using, as the input image for training, the measurement data of these various sampling patterns. For example, in a case of the rectangular sampling pattern, as shown in Fig. 15, a low-frequency region that is the measurement region in the k-space data of the correct answer image 1510 is cut out with a rectangular sampling pattern 1501, the outside thereof is subjected to zero-filling, and the k-space data 1502 is subj ected to an inverse Fourier transform to obtain an input image for training 1520. The same applies to other sampling patterns, for example, an input image for training for an elliptical shape, as shown in Fig. 16, can be created by changing the shape of the sampling pattern 1501 to a circular shape. In addition, in a case of expanding the matrix size by zero-filling after the cutout, the expansion in only the one-dimensional direction or the expansion in only the two-dimensional direction for three-dimensional data may be performed as in Application Example 2 described above, to use the low-dimensional input image for training.

The CNN is trained by using the prepared correct answer image and the input image for training. The training process is the same as the training process in Embodiment 1, and the parameters of the CNN are adjusted until a predetermined Loss function is minimized, as shown in Fig. 6. In the repetition of the training process, the DC processing (Fig. 6: S13) may be inserted, or the weighting of the Loss function with respect to the error in the measurement matrix region (processing of Embodiment 3) may be performed.

In the application of the CNN, in accordance with the sampling pattern of the image that is the correction target, the CNN of the corresponding sampling pattern is selected, and the ringing correction processing is performed. Then, the DC processing is performed, and the CNN application and the DC processing are repeated as appropriate, which is the same as in Embodiments 1 to 3.

According to the present application example, by applying the CNN for each sampling pattern of the image, more effective ringing correction can be realized.

Although the embodiments and the application examples of the present invention are described above, each processing included in the embodiments and the application examples can be combined as long as there is no technical contradiction, known processing such as filtering can be added, and such changes are also included in the present invention.

Explanation of References

1: MRI apparatus
10: imaging unit
20: computer
23: image processing unit
231: image generation unit
232: data transformation unit
233: matrix size change unit
234: ringing correction unit
235: CNN
236: combining unit (DC processing unit)

**Claims**

1. A magnetic resonance imaging apparatus (1) comprising:

   an imaging unit (10) that collects measurement data consisting of nuclear magnetic resonance signals; and one or more processors than have a function of reconstructing the measurement data at a desired reconstruction matrix size and a function of correcting ringing which occurs in a reconstructed image in a case in which a measurement matrix size of the measurement data and the reconstruction matrix size are different from each other,
   wherein the one or more processors have one or a plurality of CNNs (235) that have been trained to generate an output image in which the ringing of an input image is corrected, and are configured to generate composite data by replacing a part of k-space data obtained by performing a Fourier transform on the output image of the CNN with the measurement data before the input image of the CNN is reconstructed and generate a ringing-corrected image by performing an inverse Fourier transform on the composite data.

2. The magnetic resonance imaging apparatus according to claim 1,
   wherein the one or more processors are configured to repeat ringing correction processing via the CNN (235) and combining processing for generating composite data at least twice.

3. The magnetic resonance imaging apparatus according to claim 1,
   wherein the CNN (235) includes a CNN that has been trained by using, as training data, a correct answer image in which the ringing has not occurred and an image obtained by performing an inverse Fourier transform on k-space data in which a size of a measurement matrix region is smaller than a matrix size of the correct answer image.

4. The magnetic resonance imaging apparatus according to claim 3,
   wherein the CNN (235) has been trained by increasing a weight of an error in the measurement matrix region to be larger than a weight of an error in a region other than the measurement matrix region in a loss evaluation function during training.

5. The magnetic resonance imaging apparatus according to claim 1,
   wherein the CNN (235) includes a CNN that has been trained to obtain a ringing correction effect for an input image in which a ratio (matrix ratio) between the measurement matrix size and the reconstruction matrix size is a predetermined value.

6. The magnetic resonance imaging apparatus according to claim 5,

   wherein the CNN (235) includes a plurality of CNNs that have been trained to obtain a ringing correction effect for input images having different matrix ratios, and
   the one or more processors select at least one of the plurality of CNNs to perform ringing correction.

7. The magnetic resonance imaging apparatus according to claim 1,
   wherein the CNN (235) includes a CNN that has been trained to obtain a ringing correction effect for at least a one-dimensional direction of the input image.

8. The magnetic resonance imaging apparatus according to claim 1,

   wherein the CNN (235) includes a plurality of CNNs that have been trained to obtain a ringing correction effect for input images having different sampling patterns, for each sampling pattern of the measurement data, and
   the one or more processors are configured to select at least one of the plurality of CNNs to perform ringing correction.

9. A magnetic resonance imaging apparatus (1) comprising:

an imaging unit (10) that collects measurement data consisting of nuclear magnetic resonance signals; and one or more processors that have a function of reconstructing the measurement data at a desired reconstruction matrix size and a function of correcting ringing which occurs in a reconstructed image in a case in which a measurement matrix size of the measurement data and the reconstruction matrix size are different from each other,

wherein the one or more processors have one or a plurality of CNNs (235) that have been trained to generate an output image in which the ringing of an input image is corrected, and the CNNs include a CNN that has been trained by using, as training data, a correct answer image in which the ringing has not occurred and an image obtained performing an inverse Fourier transform on k-space data in which a size of a measurement matrix region is smaller than a matrix size of the correct answer image, and increasing a weight of an error in the measurement matrix region that to be larger than a weight of an error in a region other than the measurement matrix region in a loss evaluation function during training.

10. An image processing method of, in a case in which measurement data consisting of nuclear magnetic resonance signals collected by a magnetic resonance imaging apparatus (10) is reconstructed at a desired reconstruction matrix size, correcting ringing that occurs in a reconstructed image in a case in which a measurement matrix size of the measurement data and the reconstruction matrix size are different from each other, the image processing method comprising:

a step of applying one or a plurality of CNNs (235) that have been trained to generate an output image in which the ringing of an input image is corrected, to a correction target image;
a step of generating k-space data by performing a Fourier transform on the correction target image before the CNN is applied and a corrected image after the CNN is applied;
a step of generating composite k-space data by replacing a part of the k-space data of the corrected image with a measurement matrix region in the k-space data of the correction target image;
a step of performing an inverse Fourier transform on the composite k-space data.

11. The image processing method according to claim 10,

wherein the step of applying the CNN (235), the step of generating the k-space data, and the step of generating the composite k-space data are repeated two or more times.

12. The image processing method according to claim 10, further comprising:

a step of training the CNN (235) using, as training data, a correct answer image in which the ringing has not occurred and an image obtained by performing an inverse Fourier transform on k-space data in which a size of the measurement matrix region is smaller than a matrix size of the correct answer image,
wherein, in the step of training the CNN, a weight of an error in the measurement matrix region is increased in a loss evaluation function during training.

13. An image processing method of, in a case in which measurement data consisting of nuclear magnetic resonance signals collected by a magnetic resonance imaging apparatus (1) is reconstructed at a desired reconstruction matrix size, correcting ringing that occurs in a reconstructed image in a case in which a measurement matrix size of the measurement data and the reconstruction matrix size are different from each other, the image processing method comprising:

a step of training a CNN (235) to generate an output image in which the ringing of an input image is corrected; and
a step of applying the CNN to a correction target image,
wherein, in the step of training the CNN, the CNN is trained by using, as training data, a correct answer image in which the ringing has not occurred and an image obtained by performing an inverse Fourier transform on k-space data in which a size of a measurement matrix region is smaller than a matrix size of the correct answer image, and increasing a weight of an error in the measurement matrix region in a loss evaluation function during training.

FIG. 1

## FIG. 2

23

**IMAGE PROCESSING UNIT**

235

231

232 DATA TRANSFORMATION UNIT

233 MATRIX SIZE CHANGE UNIT

234 RINGING CORRECTION UNIT

236 COMBINING UNIT

CNN-1

CNN-2

.
.
.

CNN-n

## FIG. 3

START

CNN TRAINING STEP — S1

CNN APPLICATION STEP — S2

DC PROCESSING STEP — S3

END

# FIG. 4

FIG. 5

CORRECT ANSWER IMAGE
(COMPLEX NUMBER)

FT

CUTOUT

ZF

IFT

INPUT IMAGE
(COMPLEX NUMBER)

500

501

502

503

504

FIG. 6

START

CREATE TRAINING DATA SET — S11

i = i + 1

TRAIN CNN(i) — S12

Data Consistency PROCESSING — S13

END? — S14

No

Yes

END

# FIG. 7

EMBODIMENT 1

START

PERFORM ZERO-FILL ON
MEASUREMENT IN k-SPACE — S21

APPLY CNN IN REAL SPACE — S22

REPLACE DATA IN MEASUREMENT RANGE
AFTER CNN IS APPLIED WITH ORIGINAL
MEASUREMENT DATA IN k-SPACE
(Data Consistency PROCESSING) — S23

TRANSFORMATION INTO REAL SPACE — S24

END

# FIG. 8

MEASUREMENT IMAGE
ANY MEASUREMENT MATRIX

800

801

802

INPUT IMAGE 803

OUTPUT IMAGE 804

FT

ZF

IFT

APPLY CNN

807

806

FT

805

IFT

MAKE MEASUREMENT MATRIX REGION ZERO

FINAL OUTPUT IMAGE 808

EP 4 535 022 A2

# FIG. 9

```
              ┌──────────────┐
              │    START     │
              └──────┬───────┘
                     │
   ┌─────────────────────────────────────┐     S21
   │        PERFORM ZERO-FILL ON          │
   │   MEASUREMENT DATA    IN k-SPACE     │
   └─────────────────┬───────────────────┘
                     │                              i = i + 1
                     ●◄───────────────────────────────────────┐
                     │                                         │
   ┌─────────────────────────────────────┐     S22            │
   │        APPLY CNN(i) IN REAL SPACE    │                    │
   └─────────────────┬───────────────────┘                    │
                     │                                         │
   ┌─────────────────────────────────────┐   S23+S24          │
   │      Data Consistency PROCESSING     │                    │
   └─────────────────┬───────────────────┘                    │
                     │                        S25              │
                  ╱──┴──╲                                      │
                 ╱       ╲           No                        │
                ╱  END?   ╲──────────────────────────────────┘
                ╲         ╱
                 ╲       ╱
                  ╲──┬──╱
                     │ Yes
              ┌──────┴───────┐
              │     END      │
              └──────────────┘
```

# FIG. 10

k-SPACE (ONE-DIMENSIONAL MODEL)

REAL SPACE

Zero-filling PROCESSING
(RECTANGULAR
WAVEFORM)

FOURIER TRANSFORM

PSF
(sinc FUNCTION)

MEASUREMENT
MATRIX
SIZE: S

$kx$ [1/m]

$x$ [m]

PERIOD = R/S*2

← RECONSTRUCTION MATRIX SIZE: R →

EP 4 535 022 A2

# FIG. 11

# FIG. 12

```
           START

PERFORM ZERO-FILL ON              S31
MEASUREMENT DATA IN k-SPACE

                              i = i + 1
APPLY CNN FOR ONE-DIMENSIONAL     S32
CORRECTION IN REAL SPACE

Data Consistency                 S33
PROCESSING/TRANSFORMATION
INTO IMAGE DATA

ROTATE IMAGE                     S34

                    S35
       END?          No

       Yes
       END
```

# FIG. 13

APPLICATION EXAMPLE 2

CNN-1 FOR ONE-DIMENSIONAL CORRECTION

CNN-2 FOR TWO-DIMENSIONAL DIRECTION CORRECTION

CNN-2 FOR THREE-DIMENSIONAL DIRECTION CORRECTION

EP 4 535 022 A2

FIG. 14

SAMPLING SHAPE (k-SPACE)

2D RECTANGULAR SHAPE

2D CIRCULAR SHAPE

3D RECTANGULAR PARALLELEPIPED SHAPE

3D CYLINDRICAL SHAPE

# FIG. 15

1500

1501

1502

IFT

1510

1520

CUTOUT/
ZERO-FILLING

IFT

CNN TRAINING DATA

EP 4 535 022 A2

# FIG. 16

ELLIPTICAL SHAPE: CNN FOR
TWO-DIMENSIONAL DIRECTION CORRECTION

ELLIPTICAL SHAPE: CNN FOR
ONE-DIMENSIONAL DIRECTION CORRECTION

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2023174423 A

- JP 2021532875 A

**Non-patent literature cited in the description**

- **QIANQIAN ZHANG et al.** *Magn Reson Med.*, 2019, vol. 82, 2133-2145